(19) Europäisches Patentamt
European Patent Office
Office européen des brevets

(11) Publication number: **0 112 604**
**A1**

(12) **EUROPEAN PATENT APPLICATION**

(21) Application number: **83304342.5**

(22) Date of filing: **27.07.83**

(51) Int. Cl.³: **C 07 D 215/20**
// C07D471/04, A61K31/47

(30) Priority: **03.11.82 US 439107**

(43) Date of publication of application: **04.07.84**
**Bulletin 84/27**

(84) Designated Contracting States: **AT BE CH DE FR GB IT LI LU NL SE**

(71) Applicant: **ELI LILLY AND COMPANY, 307, East McCarty Street, Indianapolis Indiana 46285 (US)**

(72) Inventor: **Schaus, John Mehnert, 5427 North Delaware Street, Indianapolis Indiana 46220 (US)**
Inventor: **Booher, Richard Nolan, 6886 Hillcrest Court, Indianapolis Indiana 46227 (US)**

(74) Representative: **Crowther, Terence Roger et al, Erl Wood Manor, Windlesham Surrey GU20 6PH (GB)**

(54) **Novel resolved keto compounds and process.**

(57) 4aR,8aR-1-n-Propyl-6-oxodecahydroquinoline is prepared from the corresponding racemate using optically active ditoluoyltartrate acid.

EP 0 112 604 A1

ACTORUM AG

NOVEL RESOLVED KETO COMPOUNDS AND PROCESS

This invention relates to novel resolved keto compounds and the process for resolving them from the racemic mixture.

U.S. Patent Nos. 4,198,415 and 4,230,861 disclose racemic 6-oxodecahydroquinoline of the formula

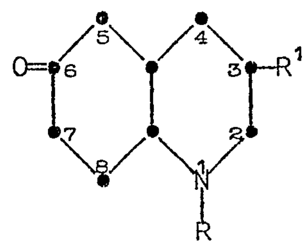

I

wherein R is hydrogen, $C_1$-$C_3$ alkyl, allyl, or benzyl; $R^1$ is hydrogen or COOZ' and Z' is $C_1$-$C_2$ alkyl, benzyl, α-methylbenzyl, or phenylethyl. The compound of formula I is reacted to form the 7-dialkylaminomethylene-6-oxodecahydroquinoline. The 7-dialkylaminomethylene compound is then cyclized with hydrazine to form a tautomeric racemic mixture of octahydropyrazolo-[3,4-g]quinolines of the formula IIa or IIb

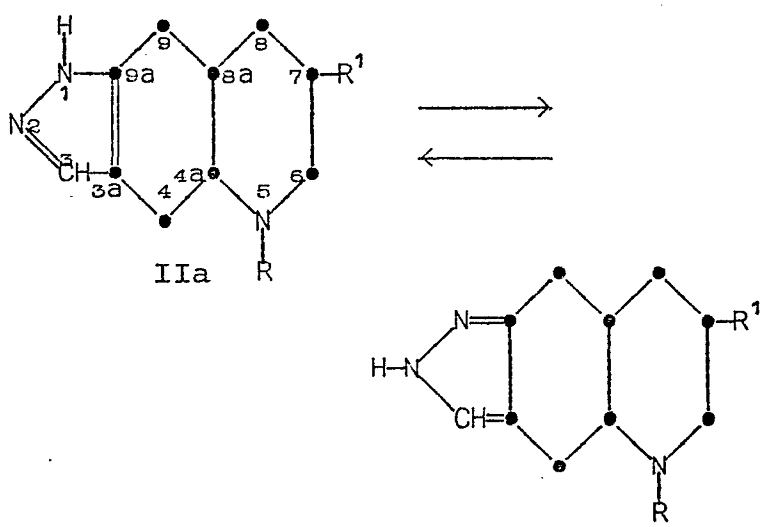

IIa

IIb

wherein R is H, $C_1$-$C_3$ alkyl, allyl or benzyl, $R^1$ is H or COOZ' and Z' is $C_1$-$C_2$ alkyl, benzyl, α-methylbenzyl, or phenylethyl. The final products of formulae IIa and IIb are useful as inhibitors of prolactin secretion and in the treatment of Parkinson's syndrome.

The present invention concerns a method of resolving the keto compound of formula I wherein $R^1$ is hydrogen. It is an advantage to resolve the keto compound into its pure stereoisomers prior to the cyclization reaction to form the compounds of formula IIa or IIb, since at least one-half of a racemic mixture is discarded during a resolution. It is clearly more economical to discard half of a starting material than half of a final product, particularly since organic reactions such as the cyclization of the ketoquinoline to a pyrazoloquinoline are never quantitative.

Thus, this invention relates to novel ketoquinolines and the process for preparing them from trans-dl-1-n-propyl-6-oxodecahydroquinoline. The two isomeric novel ketodecahydroquinoline structures are IIIa and IIIb below,

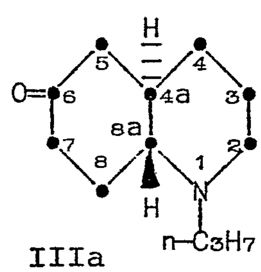
IIIa

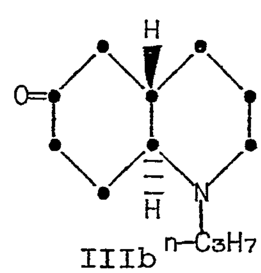
IIIb

as optically pure isomers, each free of the other, and the acid addition salts thereof.

The process of resolving trans-dl-1-n-propyl-6-oxodecahydroquinoline into its component optical isomers of the formula

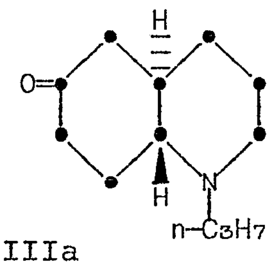

IIIa

or

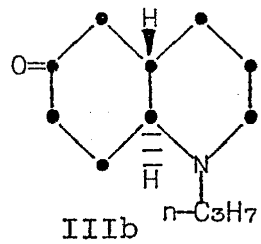

IIIb

and the acid addition salts thereof, which comprises

(a) contacting a solution of the racemate with a solution containing at least 0.5 equivalents of an optically active di-p-toluoyltartaric acid;

(b) separating the thus-formed salt of the optically active trans-1-n-propyl-6-oxodecahydroquinoline isomer and the optically active di-p-toluoyltartaric acid;

(c) treating the thus-formed salt with base;

(d) isolating the optically-active trans-1-n-propyl-6-oxodecahydroquinoline isomer therefrom; and

(e) optionally salifying the free base so obtained.

The resolution is accomplished using optically active di-p-toluoyltartaric acid as the resolving agent. When (-)-di-p-toluoyltartaric acid is reacted with a solution of the trans racemate, a

crystalline salt forms with 4aR,8aR-1-n-propyl-6-oxo-decahydroquinoline. This salt may be separated from the reaction mixture by filtration. The salt of (-)-di-p-toluoyltartaric acid with 4aS,8aS-1-n-propyl-6-oxodecahydroquinoline (the other enantiomer-IIIb) remains in the filtrate. The crystalline salt of the desired 4aR,8aR-enantiomer can be further purified by recrystallization. Optically pure 4aR,8aR-1-n-propyl-6-oxodecahydroquinoline (IIIa) is then recovered from the salt by standard procedures. In similar fashion, optically pure 4aS,8aS-1-n-propyl-6-oxo-decahydro-quinoline (IIIb) can be obtained from racemic trans-dl-1-n-propyl-6-oxodecahydroquinoline by using (+)-di-p-toluoyltartaric acid as the resolving agent.

The formula IIIa compound is preferably named as 4aR,8aR-1-n-propyl-6-oxodecahydroquinoline but is also correctly named as the trans-ℓ-isomer or the trans-(-) isomer, or as 4aα,8aβ-1-n-propyl-6-oxodecahydroquinoline.

## Choice of Resolving Agent

A number of other optically active acids were tried as potential resolving agents. These acids include (+)-tartaric acid, (-)-dibenzoyl tartaric acid, (+)-camphoric acid, (+)-10-camphorsulfonic acid, (-)-mandelic acid, (-)-malic acid, N-acetyl-L-glutamic acid and t-butyloxycarbonyl-D-phenyl glycine. These acids either failed to form a crystalline salt with dl-trans-1-propyl-6-oxodecahydroquinoline or, in those cases in which a crystalline salt did form, recrystallization

failed to provide an efficient optical purification. Thus, (-)-di-p-toluoyltartaric acid and (+)-di-p-toluoyltartaric acid appear to be unique as readily available, efficient resolving agents for trans-dl-1-n-propyl-6-oxodecahydroquinoline.

Choice of Solvent Systems

Various solvent systems were used in order to determine which gave crystalline salt of high optical purity and yield.

The following table summarizes the solvent systems employed, yield of (-)-di-p-toluoyltartrate salt based on starting trans-dl-ketone, melting point, $[\alpha]_D^{25}$ and $[\alpha]_{365}^{25}$; (both at c = 1, $CH_3OH$).

Table

| Solvent System | Tartrate salt M.P. °C. | No. Recrystal-lizations | in % yield | $[\alpha]^{25}_{D}$ | $[\alpha]^{25}_{365}$ |
|---|---|---|---|---|---|
| methylethylketone (MEK) | 146-9 | 0 | 10 | - | - |
| acetonitrile ($CH_3CN$) | 152-4 | 3 | 20 | -105.39° | -503.39° |
| methylisobutylketone (MIBK) | 156-7 | 3 | 13 | -106.39° | - |
| isopropanol | 151-2 | 3 | | -100.94° | - |
| n-propanol | 155-6 | 2 | 17 | -107.0° | -514.0° |
| MIBK/$CH_3OH$ | 158-60 | 2 | 26 | -107.8° | -515.6° |
| Ethanol | 158-8 | 2 | 20 | -106.89° | -512.29° |
| $CH_3CN/CH_3OH$ (10:1) | 157-8 | 1 | 28 | -107.6° | -515.2° |
| methanol | | 0 | 18.7 | -107.49° | |

While all of the solvents listed in the table plus methanol from Example 1 below are operative, the $C_1$-$C_3$ straight chain alkanols, such as methanol, ethanol and n-propanol are preferred, either alone or in combination with methylisobutylketone or acetonitrile. Preferably, a combination of methanol and acetonitrile is used as a solvent for the (-)-di-p-toluoyltartaric acid resolution. Methanol:acetonitrile solvent ratios from 1:4 to 1:10 have been used although these ratios are not critical as long as a finite percent of methanol is present. In addition, superior resolution is obtained at salt molarities in the neighborhood of 0.15-0.16M, although molarities outside of that range are fully operative.

Acid addition salts of compounds of formula IIIa or IIIb include salts derived from inorganic acids such as: hydrochloric acid, nitric acid, phosphoric acid, sulfuric acid, hydrobromic acid, hydriodic acid, phosphorous acid and the like, as well as salts derived from organic acids such as aliphatic mono and dicarboxylic acids, phenyl-substituted alkanoic acids, hydroxy alkanoic and alkandioic acids, aromatic acids, aliphatic and aromatic sulfonic acids. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate,

mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate, and naphthalene-2-sulfonate.

Preparing the optical isomer of an intermediate, such as 4aR,8aR-1-n-propyl-6-oxodecahydroquinoline, is always advantageous compared with resolving a final product since, in the latter procedure, the inactive isomer, constituting one-half of the starting material, is discarded. It is clearly cheaper to discard starting materials than final products. In addition, the yields for the resolution of 1-n-propyl-6-oxodecahydroquinoline are better than those for the resolution of the pyrazolo[3,4-g]quinoline derived therefrom. In addition, the resolved isomers, both 4aR,8aR and 4aS,8aS-1-n-propyl-6-oxodecahydroquinoline, are potential intermediates for the synthesis of other optically active final products; for example, other heterocyclic rings can be fused to the quinoline ring in addition to the pyrazole system [illustrated in our co-filed Application No. ~~430,834~~ (Agents ref. X-5865)] to yield novel tricyclic systems, thus obviating the necessity of developing resolution methods for each new final product.

The resolution of trans-dl-1-n-propyl-6-oxodecahydroquinoline to formula IIIa or IIIb compounds is illustrated by the following examples.

Example 1

Ten g. of (-)-di-p-toluoyltartaric acid were dissolved in 75 ml. of warm methanol. The solution was added to a solution of 5.05 g. of trans-dl-1-n-propyl-6-oxodecahydroquinoline in 15 ml. of methanol. The reaction mixture was brought to a boil and was then allowed to cool to ambient temperature. After remaining at ambient temperature overnight, crystallization was induced by the addition of seed crystals previously obtained. The crystalline tartrate salt was isolated by filtration and the filter cake washed with methanol; yield = 2.813 g. (18.7%) of a white crystalline solid comprising the (-)-di-p-toluoyltartrate of 4aR,8aR-1-n-propyl-6-oxodecahydroquinoline; $[\alpha]_D^{25}$ = -107.49° ($CH_3OH$, c = 1). Recrystallization of the salt from methanol gave 1.943 g. of the optically pure salt, $[\alpha]_D^{25}$ = -108.29° ($CH_3OH$, c = 1). The (-)-di-p-toluoyltartrate salt thus obtained was treated with dilute aqueous sodium hydroxide and the resulting alkaline solution extracted with methylene dichloride. The methylene dichloride extract was dried, concentrated and the solvent removed therefrom *in vacuo*. The resulting residue was distilled to yield a colorless oil comprising purified 4aR,8aR-1-n-propyl-6-oxodecahydroquinoline; $[\alpha]_D^{25}$ = -88.51° ($CH_3OH$, c = 1).

Other salts are prepared by dissolving the free base in ether, passing gaseous HCl through the solution or adding an ethereal solution of the acid, and isolating the ether-insoluble salt by filtration. Alternatively, a solution of the free base in a lower alkanol can be mixed with a solution of the acid in the same solvent, and the soluble salt isolated by evaporation of the solvent.

## Example 2

Following the procedure of Example 1, 48.8 g. of trans-dl-1-n-propyl-6-oxodecahydroquinoline in 200 ml. of acetonitrile were added to a warm solution of 101 g. of (-)-di-p-toluoyltartaric acid in 1 l. of acetonitrile and 300 ml. of methanol. The mixture was heated to reflux temperature and then allowed to stand at ambient temperature overnight. Seeding produced, as a first crop, 32 g. (22% yield) of 4aR,8aR-1-n-propyl-6-oxodecahydroquinoline (-)-di-p-toluoyltartrate melting at about 158-9°C.; $[\alpha]_D^{25}$ = -107.5°. (c = 1, $CH_3OH$); $[\alpha]_{365}^{25}$ = -515.5°. (c = 1, $CH_3OH$).

The above resolution was also carried out using 41.1 g. of trans-dl-1-n-propyl-6-oxodecahydroquinoline and 80.8 g. of (-)di-p-toluoyltartaric acid monohydrate in 500 ml. of anhydrous methanol. The yield was 34.5 g. (28%); $[\alpha]_D^{25}$ = -107.3°; $[\alpha]_{365}^{25}$ = -512.9% (both at c = 1, $CH_3OH$); optical purity = about 97%

## CLAIMS

1. Trans-1-n-propyl-6-oxodecahydroquinoline of the formula

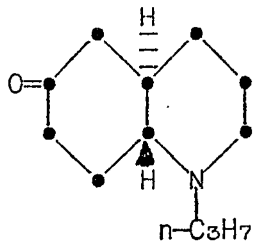

IIIa

or

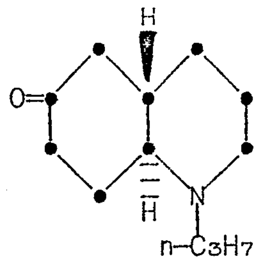

IIIb

as the optically pure isomer, and the acid addition salts thereof.

2. Optically pure 4aR,8aR-1-n-propyl-6-oxodecahydroquinoline and acid addition salts thereof.

3. The (-)-di-p-toluoyltartaric acid salt of 4aR,8aR-1-n-propyl-6-oxodecahydroquinoline.

4. 4aR,8aR-1-n-propyl-6-oxodecahydroquinoline or an acid addition salt thereof substantially free of its 4aS,8aS isomer.

5. Optically pure 4aS,8aS-1-n-propyl-6-oxodecahydroquinoline and acid addition salts thereof.

6. The (+)-di-p-toluoyltartaric acid salt of 4aS,8aS-1-n-propyl-6-oxodecahydroquinoline.

7. 4aS,8aS-1-n-propyl-6-oxodecahydroquinoline or an acid addition salt thereof, substantially free of its 4aR,8aR isomer.

8. The process of resolving trans-dl-1-n-propyl-6-oxodecahydroquinoline into its component optical isomers of the formula

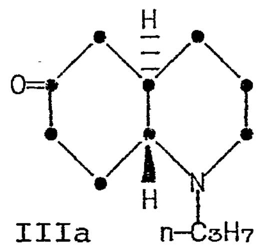

IIIa

or

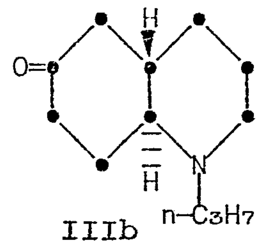

IIIb

and the acid addition salts thereof, which comprises

(a) contacting a solution of the racemate with a solution containing at least 0.5 equivalents of an optically active di-p-toluoyltartaric acid;

(b) separating the thus-formed salt of the optically active trans-1-n-propyl-6-oxodecahydroquinoline isomer and the optically active di-p-toluoyltartaric acid;

(c) treating the thus-formed salt with base;

(d) isolating the optically-active trans-1-n-propyl-6-oxodecahydroquinoline isomer therefrom; and

(e) optionally salifying the free base so obtained.

9. A process of Claim 8 in which methanol is employed as a solvent.

10. A process of Claim 8 in which the solvent is a methanol/acetonitrile mixture.

11. A process of Claim 8 in which the solvent is a methanol/methylisobutylketone mixture.

12. A process of Claim 8 or 10 in which the solvent is a 1:4 to 1:10 methanol:acetonitrile solvent mixture.

13. A process of Claim 8 in which a salt of 4aR,8aR-1-n-propyl-6-oxodecahydroquinoline is formed with (-)-di-p-toluoyltartaric acid.

14. A process of Claim 8 in which a salt of 4aS,8aS-1-n-propyl-6-oxodecahydroquinoline is formed with (+)-di-p-toluoyltartaric acid.

CLAIMS

1. The process of resolving trans-dl-1-n-propyl-6-oxodecahydroquinoline into its component optical isomers of the formula

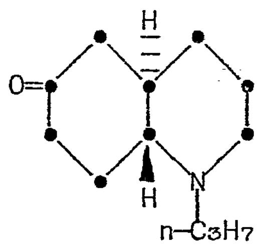

IIIa

or

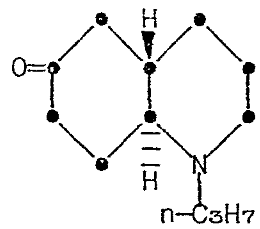

IIIb

and the acid addition salts thereof, which comprises

(a) contacting a solution of the racemate with a solution containing at least 0.5 equivalents of an optically active di-p-toluoyltartaric acid;

(b) separating the thus-formed salt of the optically active trans-1-n-propyl-6-oxodecahydroquinoline isomer and the optically active di-p-toluoyltartaric acid;

(c) treating the thus-formed salt with base;

(d) isolating the optically-active trans-1-n-propyl-6-oxodecahydroquinoline isomer therefrom; and

(e) optionally salifying the free base so obtained.

2. A process of Claim 1 in which methanol is employed as a solvent.

3. A process of Claim 1 in which the solvent is a methanol/acetonitrile mixture.

4. A process of Claim 1 in which the solvent is a methanol/methylisobutylketone mixture.

5. A process of Claim 1 or 3 in which the solvent is a 1:4 to 1:10 methanol:acetonitrile solvent mixture.

6. A process of Claim 1 in which a salt of 4aR,8aR-1-n-propyl-6-oxodecahydroquinoline is formed with (-)-di-p-toluoyltartaric acid.

7. A process of Claim 1 in which a salt of 4aS,8aS-1-n-propyl-6-oxodecahydroquinoline is formed with (+)-di-p-toluoyltartaric acid.

European Patent Office

# EUROPEAN SEARCH REPORT

0112604
Application number

EP 83 30 4342

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,X | US-A-4 230 861 (ELI LILLY)<br>* claim 2; column 14, lines 10,11 *<br>----- | 1 | C 07 D 215/20 //<br>C 07 D 471/04<br>A 61 K 31/47 |
| | | | **TECHNICAL FIELDS SEARCHED (Int. Cl. 3)** |
| | | | C 07 D 215/00 |

The present search report has been drawn up for all claims

| Place of search | Date of completion of the search | Examiner |
|---|---|---|
| THE HAGUE | 08-02-1984 | ALFARO I. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82